# EUROPÄISCHE PATENTSCHRIFT

(11) **EP 0 544 868 B1**
(45) Veröffentlichungstag und Bekanntmachung des Hinweises auf die Patenterteilung: **16.08.1995**
(21) Anmeldenummer: 92912380.0
(22) Anmeldetag: 24.06.1992
(51) Int. Cl.: F16B 39/30, F16B 33/02, A61C 8/00, A61B 17/08, F16B 25/10

(54) **BEFESTIGUNGSELEMENT**
FASTENING ELEMENT
ELEMENT DE FIXATION

(30) Priorität: 25.06.1991 CH 1875/91
(43) Veröffentlichungstag der Anmeldung: 09.06.1993
(73) Patentinhaber: Synthes AG, Chur, CH-7002 Chur (CH)
(72) Erfinder: Mathys, Robert, CH-2544 Bettlach (CH)
(74) Vertreter: Lusuardi, Werther Giovanni, Dr.
(86) Internationale Anmeldenummer: CH9200122
(87) Internationale Veröffentlichungsnummer: WO9300518

(56) Entgegenhaltungen:
- EP-A- 0 424 734
- WO-A-90/10799
- FR-A- 2 095 504
- FR-A- 2 609 757
- GB-A- 2 211 416
- US-A- 3 233 500

## Beschreibung

Die Erfindung bezieht sich auf ein Befestigungselement gemäss dem Oberbegriff des Patentanspruchs 1.
Ein Befestigungselement, in Form eines Bolzens, ist bereits aus der DE-C2 2.807.364 bekannt. Dieses bekannte, als Knochenschraube konzipierte Befestigungselement besteht aus einem gewindelosen Mittelteil an den sich im oberen, proximalen Teil und im unteren, distalen Teil des Schraubenschaftes je ein Gewinde mit verschiedenem Durchmesser und voneinander verschiedener, jedoch konstanter Steigung anschliesst. Es ist somit notwendig, vor dem Setzen der Knochenschraube, in beiden miteinander zu verbindenden Knochenfragmenten im Durchmesser unterschiedliche Bohrungen mit entsprechenden Gewinden mit unterschiedlichen Durchmessern, jedoch konstanter Steigung zu schneiden. Wird für den proximalen Teil des Schaftes ein Gewinde mit geringerer Steigung verwendet, so erfolgt beim Eindrehen dieser bekannten Knochenschraube eine Annäherung der beiden Knochenfragmente und eine entsprechende Kompression der Bruchfläche. Nachteilig bei diesem bekannten, dem Prinzip zweier Gewinde mit verschiedener Steigung gehorchenden Befestigungselementes ist der gewindelose Mittelteil und die konstante Steigung der beiden unterschiedlichen proximalen und distalen Gewinde, welche bloss eine Kompression zwischen zwei losen Knochenfragmenten erlaubt, nicht jedoch eine lokale intraossäre Druckerzeugung wie sie für eine ganze Reihe von Applikationen erwünscht wäre.
Beim bekannten Befestigungselement fehlt somit ebenso wie bei den üblichen Knochenschrauben eine im proximalen Bereich differenziert wirkende Kraft, so dass es im proximalen Bereich immer wieder zu einer Zone geringerer Knochendichte kommt, welche eine verminderte Stabilität des Implantates zur Folge haben.
Schliesslich ist aus der FR 2.095.504 NATIONAL FORGE COMPANY, von welcher der Anspruch 1 ausgeht, ein Stopfen für einen Druckbehälter bekannt, welcher im hinteren Teil eine kleinere Gewindesteigung aufweist als im vorderen Teil. Es handelt sich um einen Schraubverschluss, bei dem ein kompressibler Schraubteil in eine mit einem abweichenden Gewinde versehene inkompressible Öffnung eingeschraubt wird, so dass zwischen den beiden Gewinden ein Spiel besteht. Durch Anwendung von Druck, wie er beispielsweise in einer Druckflasche entsteht, wird dann das überdimensionierte Gewinde des kompressiblen Schraubteils komprimiert und das Spiel zwischen den beiden Gewinden aufgehoben. Ein solcher Schraubteil ist für die Knochenfixation jedoch ungeeignet.

Hier will die Erfindung Abhilfe schaffen. Der Erfindung liegt die Aufgabe zugrunde, ein gewindetes Befestigungselement zu schaffen, welches selbstschneidend oder in einem vorgeschnittenen Gewinde mit konstanter Steigung in ein mindestens geringfügig elastisch komprimierbares Material, beispielsweise Knochenmaterial oder Weichholz, eingedreht werden kann und dabei, z.B. bei Knochen eine lokal differenzierte, intraossäre Druckerzeugung bewirkt, welche mit den biologischen Erfordernissen bezüglich Krafteinleitung und der lokalen Belastung des Materials in Übereinstimmung gebracht werden kann.

Die Erfindung löst die gestellte Aufgabe mit einem Befestigungselement, welches die Merkmale des Anspruchs 1 aufweist.

Die durch die Erfindung erreichten Vorteile sind im wesentlichen darin zu sehen, dass dank der erfindungsgemässen in proximaler Richtung, vorzugsweise kontinuierlich, abnehmenden Steigung des Gewindes bei Knochen eine lokal differenzierte, intraossäre Druckerzeugung stattfindet, welche gemäss dem bekannten Wolffschen Gesetz zu einem gesteuerten Knochenwachstum entlang der Belastungslinien führt.
Die dadurch erzielte gute Knochendichte in diesem heiklen Bereich führt zu einer stabilen Integration des Implantates.

Ein Ausführungsbeispiel der Erfindung, welches zugleich das Funktionsprinzip erläutert, ist in der Zeichnung dargestellt und wird im folgenden näher beschrieben.
Fig. 1 stellt einen Querschnitt durch das erfindungsgemässe Befestigungselement dar;
Fig. 2 stellt einen Querschnitt durch das erfindungsgemässe Befestigungselement gemäss Fig. 1 dar, dessen linken Bildhälfte eine erste Phase beim Eindrehen und dessen rechte Bildhälfte die Schlussphase nach erfolgtem Eindrehen in den Knochen zeigt;
Fig. 3 stellt einen partiellen Querschnitt des erfindungsgemässen Befestigungselementes mit den gemäss Fig. 2 auftretenden Kräften dar;
Fig. 4 stellt einen Querschnitt durch ein erfindungsgemässes Befestigungselement mit konischer Kopfpartie dar;
Fig. 5 stellt einen Querschnitt eines erfindungsgemässen Befestigungselement in Form eines Dentalimplantates mit Konus dar, bei welchem das Gewinde gegen den Konus hin eine differenzierte, abnehmende Steigung aufweist;
Fig. 6 stellt einen partiellen Querschnitt durch das Gewinde des Befestigungselementes gemäss Fig. 4 dar.

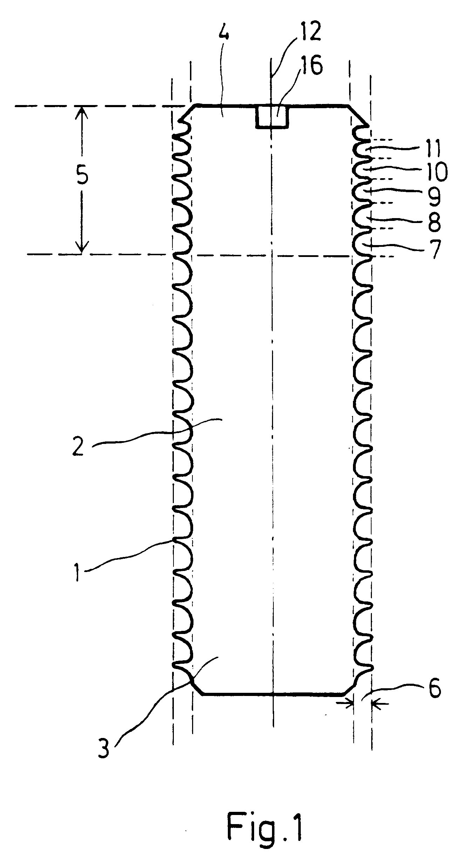

Das in Fig. 1 dargestellte, erfindungsgemässe Befestigungselement besteht im wesentlichen aus einem, ein Gewinde 1 tragenden kreiszylindrischen Schaft 2, einem vorderen Ende 3, einem hinteren Ende 4 und der Rotationsachse 12. Im hinteren Ende des Schaftes 2 ist ein Schlitz oder eine sechskantige Vertiefung 16 vorgesehen, in welche ein geeignetes Instrument (Schraubenzieher, Sechskantschlüssel) eingeführt werden kann um das Befestigungselement ein- und ausdrehen zu können. Das Gewinde 1 ist bei dieser bevorzugten Ausführungsform selbstschneidend und durchgehend über den gesamten Bereich des Schaftes 2 ausgebildet. Die Gewindetiefe ist mit 6 bezeichnet. Währenddem das Gewinde 1 im Bereich des vorderen Endes 3 eine konstante Steigung von z.B. einem Millimeter pro Gewindegang aufweist, nimmt die Steigung im Teilbereich 5 des hinteren Endes 4 von Gewindegang zu Gewindegang ab. Gewindegang 7 weist noch eine Steigung von 0,95 mm, Gewindegang 8 von 0,9 mm, Gewindegang 9 von 0,85 mm, Gewindegang 10 von 0,80 mm und Gewindegang 11 von 0,75 mm auf. Dadurch kann lokal ein differenzierter intraossärer Druck erzeugt werden, indem das elastische Material, in welches das Befestigungselement eingedreht wird, in die abnehmende Gewindesteigung hineingepresst wird.

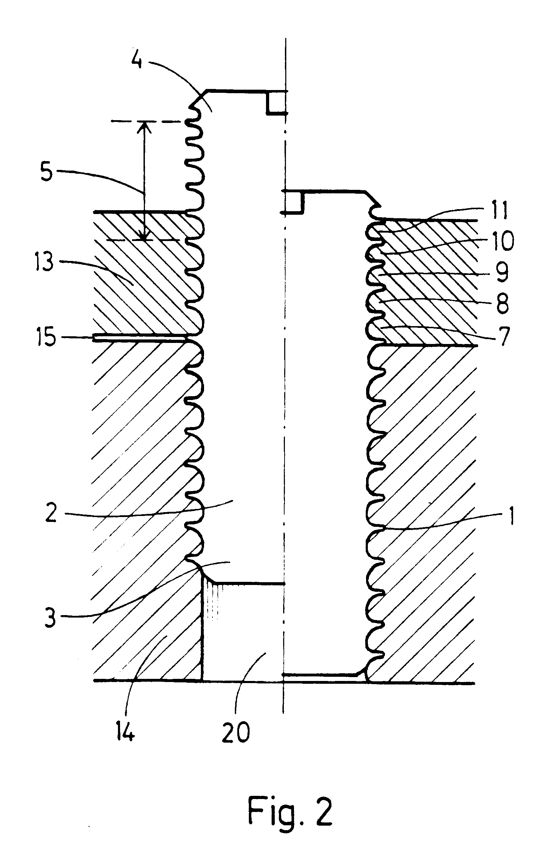

Aus Fig. 2 ist eine Applikation des erfindungsgemässen Befestigungselementes ersichtlich, bei der zwei Elemente gegeneinander gepresst werden sollen. Sie eignet sich insbesondere zur Fixation eines Knochentransplantates in der Knochenchirurgie, kann jedoch auch für eine Verbindung anderer Materialien, wie beispielsweise Weichholz, angewendet werden.
In der linken Bildhälfte ist der Zustand dargestellt, wie er sich nach dem Eindrehen des mit konstanter Gewindesteigung ausgebildeten vorderen Endes 3 des Schaftes 2 durch das vorgebohrte Kernloch 20 im Kiefer 14 und durch das Transplantat 13 ergibt. Der hier vorhandene, unerwünschte Spalt 15 von 0,20 mm zwischen Transplantat 13 und Kieferknochen 14 bleibt bis zu dieser Eindrehphase erhalten. Sobald nun der mit abnehmender Steigung des Gewindes 1 augebildete Teilbereich 5 des hinteren Endes 4 in das Transplantat 13 eingedreht wird, ergibt sich wegen der im Bereich des Kieferknochens 14 vergleichsweise kleineren Gewindesteigung auch ein unterschiedlicher Betrag für den zurückgelegten Weg des Befestigungselementes, was nach Eindrehung der vier Gewindegänge 7 - 10 (mit einer Gesamtdifferenz von 0,20 mm) zu einer vollständigen Schliessung des 0,20 mm breiten Spaltes 15 führt und schliesslich - wie in der rechten Bildhälfte dargestellt - nach Eindrehen des letzten Gewindeganges 11 zu einer intraossären Kompression führt, welche - dank der kontinuierlich von Gewindegang zu Gewindegang abnehmenden Steigung des Gewindes 1 im Teilbereich 5 - gegen das hintere Ende 4 hin graduell zunimmt, wie dies biologisch wünschenswert ist.

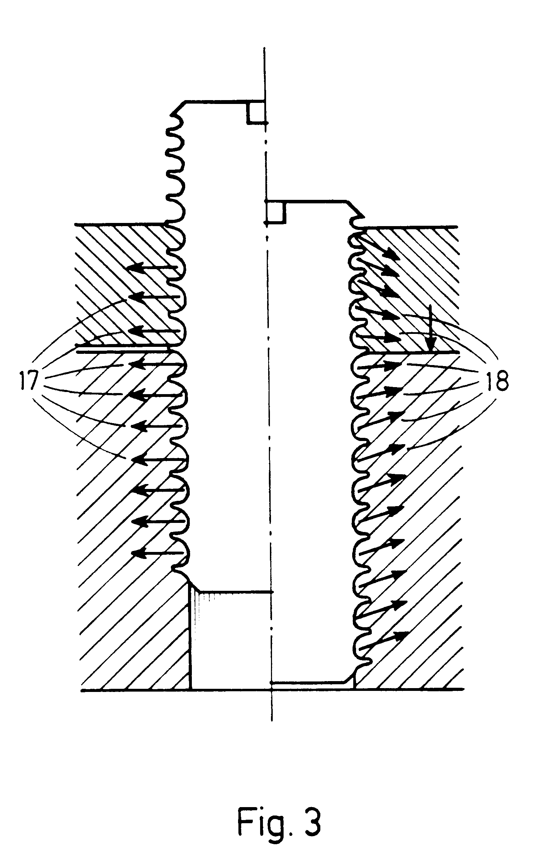

Die beim Übergang von der intermediären Eindrehphase in der linken Bildhälfte zur Schlussphase in der rechten Bildhälfte von Fig. 2 auf der Höhe der einzelnen Gewindegänge auftretenden Kräfte ist in Fig. 3 durch die Pfeile 17 (intermediäre Eindrehphase ohne Kompression) und Pfeile 18 (Schlussphase mit Kompression) angedeutet.

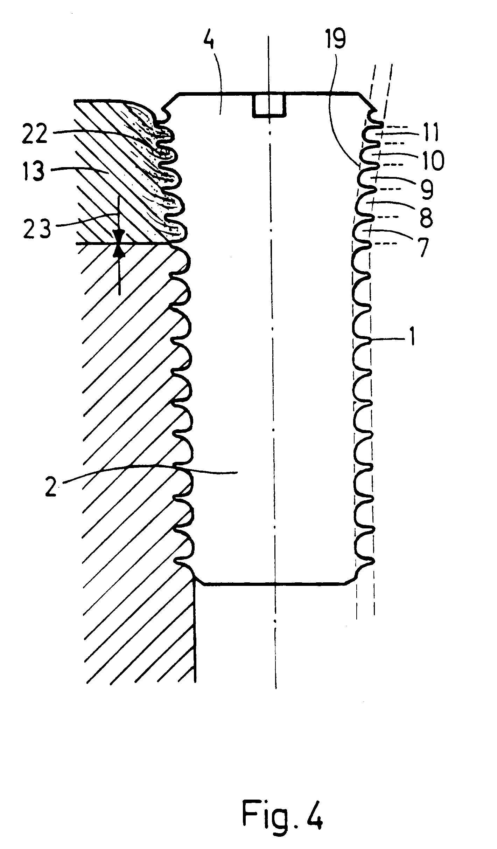

Bei einer bevorzugten Ausführungsform des erfindungsgemässen Befestigungselementes, wie in Fig. 4 gezeigt, ist das hintere Ende 4 des Schaftes 2 konisch ausgebildet, wobei sich der Konus 19 gegen das hintere Ende 4 hin verbreitert. Im kreiszylindrischen Bereich des Schaftes 2 weist das Gewinde 1 eine konstante Steigung von 1,0 mm auf, währenddem im sich erweiterenden Bereich des Konus 19 die Steigung des Gewindes 1 gegen das hintere Ende 4 kontinuierlich abnimmt.

In der linken Bildhälfte ist dargestellt wie das vom Gewinde 1 erfasste Materialvolumen 22 des Knochentransplantates 13 beim Eindrehen der am Konus 19 angebrachten Gewindegänge 7 - 11 mit abnehmender Steigung in die volumenmässig kleineren Gewindegänge 7 - 11 eingepresst wird. Dadurch entsteht ein lokal differenzierter Druck, welcher gleichzeitig in eine axial wirkende Kraft (angedeutet durch den Pfeil 23) umgewandelt wird.
Diese teilweise konische Ausführungsform des erfindungsgemässen Befestigungselementes ergibt gegenüber der kreiszylindrischen Ausführung nach Fig. 1 - 3 eine zusätzliche Sicherheit in der Fixation.

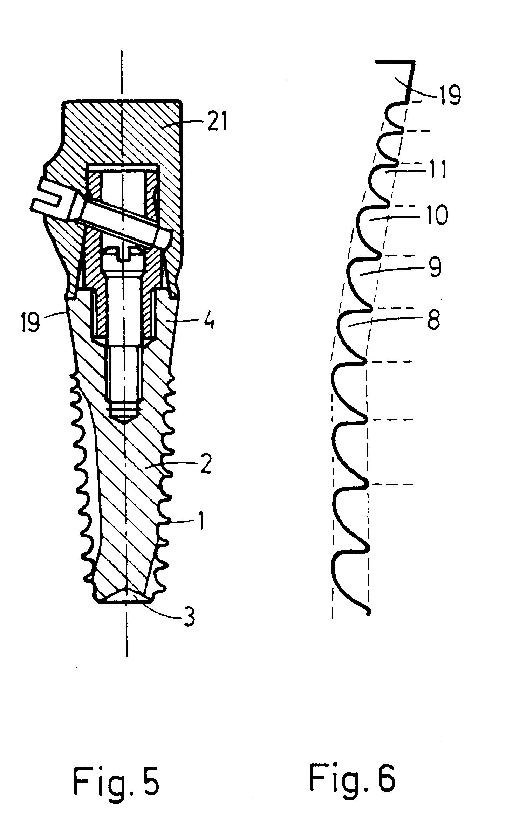

Bei einer weiteren bevorzugten Applikationsform nach Fig. 5 ist das erfindungsgemässe Befestigungselement als Dentalimplantat ausgebildet. Auf dem hinteren Ende 4 sind hier für Dentalimplantate übliche Suprastrukturelemente 21 angebracht. Im übrigen ist der Schaft 2 mit dem Gewinde 1 gegen das vordere Ende 3 hin konisch gestaltet. Das Gewinde 1 reicht vom vorderen Ende 3 bis zum als Konus 19 ausgebildeten hinteren Ende 4. Der Konus 19 selbst ist gewindefrei, wie dies für supragingivale Implantate üblich ist.

Bei einer in Fig. 6 teilweise dargestellten Ausführungsform für subgingivale Dentalimplantate ist der Konus 19 teilweise mit Gewindegängen 8 - 11 ausgestattet, deren Steigung sich gegen proximal verringert.

Das erfindungsgemässe Befestigungselement kann aus üblichen, für Schrauben geeignete Metalle gefertigt werden. Bei einer Verwendung als Dentalimplantat wird jedoch vorzugsweise Reintitan verwendet.

## Patentansprüche

1. Befestigungselement mit einem, ein Gewinde (1) mit unterschiedlicher Steigung tragenden Schaft (2), einem vorderen Ende (3) und einem hinteren Ende (4), wobei das Gewinde (1) durchgehend über den gesamten Bereich des Schaftes (2) ausgebildet ist und im Bereich des hinteren Endes (4), mindestens in einem Teilbereich (5) davon, eine kleinere Steigung aufweist als im Bereich des vorderen Endes (3), dadurch gekennzeichnet, dass die Tiefe (6) des Gewindes mit abnehmender Steigung des Gewindes (1) abnimmt.

2. Befestigungselement nach Anspruch 1, dadurch gekennzeichnet, dass das Gewinde (1) mindestens in einem Teilbereich (5) eine von Gewindegang zu Gewindegang vom vorderen (3) zum hinteren Ende (4) abnehmende Steigung aufweist.

3. Befestigungselement nach Anspruch 1, dadurch gekennzeichnet, dass das Gewinde (1) im Bereich des vorderen Endes (3) eine konstante Steigung aufweist.

4. Befestigungselement nach einem der Ansprüche 1 - 3, dadurch gekennzeichnet, dass der Schaft (2) kreiszylindrisch ausgebildet ist.

5. Befestigungselement nach einem der Ansprüche 1 - 3, dadurch gekennzeichnet, dass der Schaft (2) mindestens im Bereich des hinteren Endes (4) konisch ausgebildet ist, und sich der Konus gegen das hintere Ende (4) verbreitert.

6. Befestigungselement nach Anspruch 5, dadurch gekennzeichnet, dass der Schaft (2) durchgehend konisch ausgebildet ist.

7. Befestigungselement nach einem der Ansprüche 1 bis 6, dadurch gekennzeichnet, dass die Steigung des Gewindes (1) mindestens in einem Teilbereich (5) pro Gewindegang um 0,04 bis 0,06 mm, vorzugsweise um 0,045 bis 0,055 mm abnimmt.

8. Befestigungselement nach einem der Ansprüche 1 bis 7, dadurch gekennzeichnet, dass die Steigung des Gewindes (1) mindestens in einem Teilbereich (5) eine kontinuierlich vom vorderen (3) zum hinteren Ende (4) abnehmende Steigung aufweist.

9. Befestigungselement nach einem der Ansprüche 1 bis 8, dadurch gekennzeichnet, dass die Steigung des Gewindes (1) über den gesamten Bereich des Schaftes (2) eine vorzugsweise um den Betrag von 0,01 bis 0,02 mm pro Gewindegang abnehmende Steigung aufweist.

10. Befestigungselement nach einem der Ansprüche 1 bis 9, dadurch gekennzeichnet, dass das Gewinde (1) selbstschneidend ausgebildet ist.

11. Verwendung des Befestigungselementes nach einem der Ansprüche 1 - 10 für osteosynthetische Befestigungsvorrichtungen, insbesondere für Befestigungsvorrichtungen zur Fixation von kieferchirurgischen Transplantaten und von Mehrfragmentbrüchen.

12. Verwendung des Befestigungselementes nach einem der Ansprüche 1 - 10 für ein Dentalimplantat.

## Claims

1. A fastener with a shaft (2) equipped with a thread (1) with variable pitch, a front end (3) and a rear end (4), whereby the thread (1) extends over the entire area of the shaft (2), and has a smaller pitch in the area of the rear end (4), at minimum in a section (5) thereof, than in the area of the front end (3), characterized in that the depth (6) of the thread (1) is reduced with diminishing pitch of the thread.

2. A fastener according to claim 1, characterized in that the thread (1) at least in a section (5) has a pitch that diminishes from convolution to convolution from the front end (3) to the rear end (4).

3. A fastener according to claim 1, characterized in that the thread (1) in the area of the front end (3) has a constant pitch.

4. A fastener according to one of the claims 1 - 3, characterized in that its shaft (2) is shaped in the form of a circular cylinder.

5. A fastener according to one of the claims 1 - 3, characterized in that the shaft (2), at least in the area of the rear end (4), is conically shaped, and that the cone widens toward the rear end (4).

6. A fastener according to claim 5, characterized in that the shaft (2) is shaped conically throughout.

7. A fastener according to one of the claims 1 - 6, characterized in that the depth (6) of the thread (1) diminishes by 0,04 to 0,06 mm per thread convolution, and preferably by 0,045 to 0,055 mm, at least in a section (5).

8. A fastener according to one of the claims 1 - 7, characterized in that the thread (1), at least in a section (5), shows a continuously diminishing pitch from the front end (3) to the rear end (4).

9. A fastener according to one of the claims 1 - 8, characterized in that the thread (1) shows a pitch diminishing preferably by 0,01 to 0,02 mm per thread convolution, over the entire area of the shaft (2).

10. A fastener according to one of the claims 1 - 9, characterized in that the thread (1) is designed in such a way as to make its own incision.

11. Use of the fastener according to one of the claims 1 - 10 for osteosynthetic fastening devices, especially for fastening devices for securing jaw surgery transplants and multiple fragment breaks.

12. Use of the fastener according to one of the claims 1 - 10 for dental implants.

## Revendications

1. Elément de fixation comportant une tige (2) portant un filet (1) à pas variable, une extrémité avant (3) et une extrémité arrière (4), le filet (1) étant conformé de manière continue sur la totalité de la région de la tige (2) et présentant dans la région de l'extrémité arrière (4), au moins dans une région partielle (5) de celle-ci, un pas plus petit que dans la région de l'extrémité avant (3), caractérisé en ce que la profondeur (6) du filet diminue en même temps que le pas du filet (1).

2. Elément de fixation selon la revendication 1, caractérisé en ce que le filet (1) présente au moins dans une région partielle (5) un pas qui diminue d'une spire à l'autre entre l'extrémité avant (3) et l'extrémité arrière (4).

3. Elément de fixation selon la revendication 1, caractérisé en ce que le filet (1) présente un pas constant dans la région de l'extrémité avant (3).

4. Elément de fixation selon l'une quelconque des revendications 1-3, caractérisé en ce que la tige (2) est de conformation cylindrique circulaire.

5. Elément de fixation selon l'une quelconque des revendications 1-3, caractérisé en ce que la tige (2) est conformée en cone au moins dans la région de l'extrémité arrière (4), le cône s'élargissant vers l'extrémité arrière (4).

6. Elément de fixation selon la revendication 5, caractérisé en ce que la tige (2) est conformée comme cône continu.

7. Elément de fixation selon l'une quelconque des revendications 1 à 6, caractérisé en ce qu'au moins dans une région partielle (5), le pas du filet (1) diminue de 0,04 à 0,06 mm, de préférence de 0,045 à 0,055 mm par spire.

8. Elément de fixation selon l'une des quelconque revendications 1 à 7, caractérisé en ce qu'au moins dans une région partielle (5), le pas du filet (1) présente un pas diminuant de manière continue depuis l'extrémité avant (3) jusqu'à l'extrémité arrière (4).

9. Elément de fixation selon l'une quelconque des revendications 1 à 8, caractérisé en ce que sur toute la région de la tige (2), le pas du filet (1) présente un pas qui diminue de préférence de la valeur de 0,01 à 0,02 mm par spire.

10. Elément de fixation selon l'une quelconque des revendications 1 à 9, caractérisé en ce que le filet (1) est auto-taraudeur.

11. Utilisation de l'élément de fixation selon l'une quelconque des revendications 1-10 pour des dispositifs de fixation en ostéosynthèse, en particulier pour des dispositifs de fixation en vue de la fixation d'implants en chirurgie de la mâchoire et de l'immobilisation de fractures en plusieurs fragments.

12. Utilisation de l'élément de fixation selon l'une quelconque des revendications 1-10 pour un implant dentaire.
